# EUROPEAN PATENT APPLICATION

(11) **EP 2 241 576 A1**
(43) Date of publication of application: **20.10.2010**
(21) Application number: 09158179.3
(22) Date of filing: 17.04.2009
(51) Int. Cl.: C07K 16/28, C07K 16/30, C07K 16/46

(54) **Use of trifunctional bispecific antibodies for the treatment of tumors associated with CD133+/EpCAM+ cancer stem cells**

(71) Applicant: TRION PHARMA GMBH, 80807 München (DE)
(72) Inventor: Lindhofer, Horst, 80639 München (DE)
(74) Representative: Behnisch, Werner

(57) **Abstract**

Use of a trifunctional bispecific antibody having the following properties of: a) binding to a T cell; b) binding to EpCAM as tumor-associated antigen on a tumor cell wherein the tumor cell additionally carries the membrane bound glycoprotein CD133; c) binding by its Fc portion to Fc receptor-positive cells for the preparation of a pharmaceutical composition for the destruction of cancer stem cells carrying the tumor-associated antigen EpCAM and the membrane bound glycoprotein CD133 in a population of patients suffering from a tumor.

## Description

The invention relates to the use of a pharmaceutical preparation containing trifunctional bispecific antibodies for the destruction of tumor cells associated with tumors in a population of patients carrying on said tumor cells EpCAM as tumor-associated antigen and additionally the membrane bound glycoprotein CD133 as a marker protein for cancer stem cells in order to treat said tumors.

Solid tumors are an enormous cancer burden and a major therapeutic challenge. The cancer stem cell (CSC) hypothesis provides a convincing cellular mechanism to account for the therapeutic refractoriness and the dormant behaviour exhibited by many of these tumors. It has been reported that only a small fraction of tumour cells in a malignancy have the capacity to propagate the tumor upon transplantation into immune-compromised mice. Those cells are termed CSC or "tumor-initiating cells" and can be selected based on the expression of cell surface markers associated with immature cell types (e.g. CD133). Thus, CSCs have been defined in analogy to normal cells, as cells that have the capacity to self-renew, meaning undergo cell division that allow generation of more CSCs and give rise to the variety of differentiated cells found in malignancy (Clark et al., Cancer Res 66: 9339, 2006).

If CSCs are the only cells in cancer disease with the ability to expand and promote tumor growth, and more importantly with the capacity to metastasize, then novel cancer therapies should target the CSC population. Recently, there is some evidence that CSCs are relative resistant to chemo- and radiotherapeutic approaches. In haematological malignancies it appears that most CSCs are in a non-dividing quiescent state and therefore much less sensitive to classical anti-proliferative chemotherapeutic regimens. It has been reported that CSCs often showed high expression of drug transporters indicating that these cells are better capable of dealing with chemotoxic agents (Dean et al., Nat Rev Cancer 5: 275, 2005).

Hence, the current conventional treatments are unlikely to effectively kill CSC population. In addition, for colon carcinomas it was reported that CSCs showed more chemoresistance than differentiated tumor cells from the same patient (Todaro et al., Cell Stem Cell 1: 389, 2007). Furthermore, in glioblastoma it was evident that after irradiation the fraction of CD133+ cells actually increased after treatment which possibly reflects an intrinsic difference in radiosensitivity (Bao et al., Nature 444: 756, 2006). The CD133 membrane glycoprotein represents a prominent CSC marker that has been previously demonstrated to be capable of identifying a cancer initiating subpopulation in brain tumors, melanoma and EpCAM⁺ solid tumors (Hermann et al.,Cell Stem Cell 2007, O'Brien et al., Nature 2007, Ricci-Vitiani et al., Nature 2007; Eramo et al., Cell Death Differ 2007). The co-localization of EpCAM and CD133 on CSC obviously give rise to new treatment modalities to target these CSCs directly and would be of immense therapeutic benefit.

Exemplarily, malignant ascites (MA) was investigated by the inventors in more detail to find a new treatment method for the destruction of cancer stem cells.

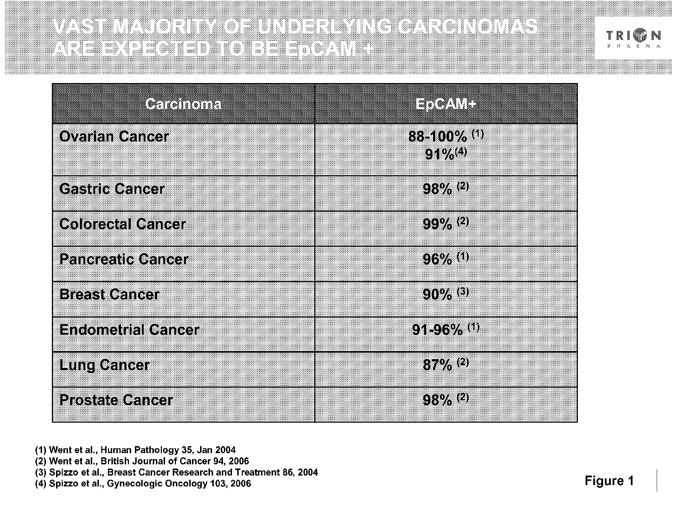

Malignant ascites may be caused by a plurality of primary tumors such as e.g. breast cancer, ovarian carcinoma or the gastrointestinal carcinomas (figure 1). Although ascites is detected in a high percentage of patients already during the first manifestation of a tumor disease, it is an indication of a progressive disease.

The present options for a therapy of ascites include puncture, local chemotherapy, or diuretic treatment. All these options have dramatic disadvantages; thus, puncture only leads to a short-term alleviation and has to be repeated after 9.5 days on average (Mackey et al., J. Pain Symptom Manage, 19:193, 2000). Chemotherapy, however, can only be successful in patients who have not already developed chemotherapy-resistant tumor cells which unfortunately is often the case. In this respect, there is an enormous need for an improvement of the clinical treatment options in the case of ascites.

EP 1 315 520 suggested to treat patients with malignant ascites and/or plural effusion with trifunctional bispecific antibodies having the following properties:
a) binding to a T cell;
b) binding to at least an antigen on a tumor cell associated with malignant ascites and/or pleural effusion;
c) binding, by its Fc portion to Fc receptor-positive cells

While evidence for elimination of tumor cells bearing EpCAM as tumor-associated antigen associated with malignant ascites and pleural effusion have been shown, it is also known that tumor patients suffering from malignant ascites have to be divided into two different subpopulations. One population is known to carry only EpCAM positive tumor cells while the other sub-population either additionally or solely contains in the tumor patient's peritoneal fluid tumor cells with EpCAM as tumor-associated antigen and additionally the membrane bound glycoprotein CD133 so that these tumor cells can be characterized as cancer stem cells.

The population carrying only EpCAM positive tumor cells may be well treated by the methods of EP 1 315 520. However, as explained above, cancer stem cells are highly resistant against various therapeutic measures, and it was completely uncertain and unknown whether or not the second group of tumor patients characterized by EpCAM+/CD133+ cancer stem cells can be efficiently treated by trifunctional bispecific antibodies with the characteristics described above. There had been a high uncertainty whether patients carrying cancer stem cells being EpCAM positive/CD133 positive will be successfully treatable or be resistant against the trifunctional bispecific antibody therapy.

If one assumes that a population of tumor patients with tumor cells being EpCAM+/CD133+ is resistant to conventional therapy, one had to screen the population of patients before treatment whether or not the tumor cells carry the CD133 marker protein identifying these tumor cells as cancer stem cells or not. More aggressive therapeutic methods, e.g. higher doses of administrations of said antibodies combined with chemotherapy, would then be necessary to efficiently treat also this sub-population of patients carrying CD133 as cancer stem cell marker. On the other hand, if one positively knows of the efficacy of the therapeutic approach of using trifunctional bispecific antibodies with the elements defined above, the medical doctor can save time and money omitting additional screening tests and may proceed more rapidly and with higher probability of success with the therapeutic measurements.

It is of particular importance to have in mind that the state of the art strongly suggests that tumor cells associated with EpCAM tumor antigen as well as CD133 membrane glycoprotein identifying these cells as putative cancer stem cells are either recalcitrant or hard to be treatable by conventional therapeutic methods including monoclonal antibody therapy.

It is an object of the invention to provide a novel therapy for tumors associated with a tumor cell population carrying both, EpCAM tumor antigen as well as CD133 membrane glycoprotein identifying these cells as putative cancer stem cells. It is a further object of the invention to avoid pre-screening of patients with tumors before therapy in order to avoid selecting a sub-population carrying only EpCAM associated tumor antigens and a sub-population carrying EpCAM positive/CD133 positive tumor cells which might be treatment-resistant or to be treated by different therapeutic methods

These problems have been found to be solved by antibodies facilitating a cooperation of different classes of immune cells selected from trifunctional bispecific antibodies having the following properties of:
a) binding to a T cell;
b) binding to EpCAM as tumor-associated antigen on a tumor cell wherein the tumor cell additionally carries the membrane bound glycoprotein CD133;
c) binding by its Fc portion to Fc receptor-positive cells
   with a composition to destruct tumor cells in a population of patients carrying on said tumor cell EpCAM as tumor-associated antigen and additionally the membrane bound glycoprotein CD133.

The present invention also provides a method for the treatment of humans or animals suffering from tumor diseases by contacting the humans' or animals' bodies containing these tumor cells with a pharmaceutically effective amount of trifunctional bispecific antibodies having the following properties of:
a) binding to a T cell;
b) binding to EpCAM as tumor-associated antigen on a tumor cell wherein the tumor cell additionally carries the membrane bound glycoprotein CD133;
c) binding by its Fc portion to Fc receptor-positive cells
   for the preparation of a pharmaceutical composition for the destruction of tumor cells in a population of patients carrying on said tumor cells EpCAM as tumor-associated antigen and additionally the membrane bound glycoprotein CD133.

One well described antibody useful in the presently claimed invention is the trifunctional anti-EpCAM x anti-CD3 antibody catumaxomab which has now herein been shown to provide the capacity to eliminate CSCs from the body. While it has been recently shown that catumaxomab efficiently eliminates tumor cells from the peritoneal fluid of patients suffering from malignant ascites (MA) as demonstrated in a phase I/II trial (Burges et al., Clin Cancer Res, 13:3899, 2007) and a pivotal phase II/III trial (Jager et al., ASCO 2007; Parsons et al., ASCO 2008) the invention now focuses on the surprising effect of elimination of CD133⁺ EpCAM⁺ CSCs from a sub-group of patients suffering from tumors which are characterized by the tumor-antigen EpCAM and the glycoprotein CD133 identifying these tumor cells as cancer stem cells being known to be hardly treatable.

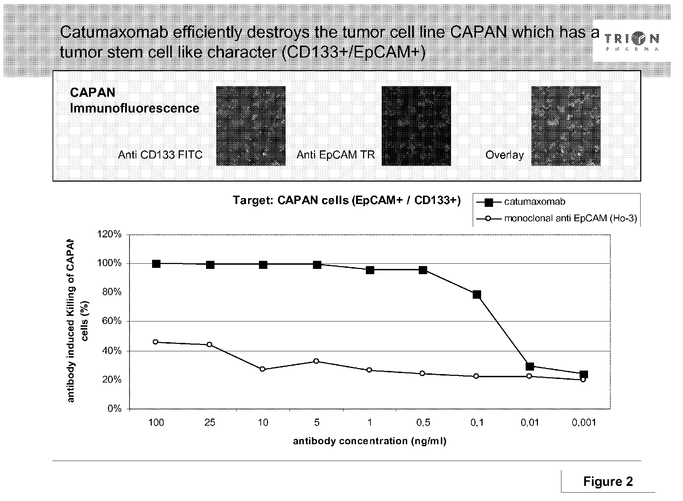

As can be seen from the enclosed figure 2, only the trifunctional bispecific antibody catumaxomab was able to efficiently kill tumor cell lines associated with CD133+/EpCAM+ having tumor stem cell like character. Using a conventional monoclonal anti-EpCAM antibody does not result in an efficient destruction of CD133+/EpCAM+ tumor stem cells. This clearly provides evidence for the surprising and non-expectable effect of trifunctional bispecific antibodies like catumaxomab in the efficient destruction of tumor stem cells characterized by CD133+/EpCAM+ antigens.

The present invention also focuses on, but is not limited to the elimination of tumor cells in those sub-groups of patients suffering from malignant ascites and pleural effusion and carrying on their tumor cells the tumor-antigen EpCAM and the glycoprotein CD133 identifying these tumor cells as cancer stem cells being known to be resistant to various therapeutic measures.

As the primary target of catumaxomab and any other trifunctional antibody as described herein is a tumor stem cell characterized by EpCAM as tumor-associated antigen and additionally the membrane bound glycoprotein CD133, it is evident from the results of the experimental data presented herein to confer the positive treatment results of MA patients to other tumor patients with cancer stem cells defined by EpCAM as tumor-associated antigen and additionally the membrane bound glycoprotein CD133.

Further embodiments of the present invention may be seen from the accompanying claims as well as the following description. It has to be pointed out that the invention is not limited to the preferred embodiments and the Examples mentioned below. In contrast, those skilled in the art in the frame of their technical knowledge may modify the invention in the scope of the accompanying claims in connection with the description without departing from the invention.

The accompanying Figures serve to further illustrate the present invention. The Figures show:
Figure 1: Vast majority of underlying carcinomas are expected to be EpCAM+.
Figure 2: Catumaxomab effiently destroys the tumor cell line CAPAN which has a tumor stem cell like character (CD133+/EpCAM+).
Figure 3: Catumaxomab destroys putative cancer stem cells (EpCAM+/CD133+) from malignant ascites in an autologous setting in vitro I.
Figure 4: Catumaxomab destroys putative cancer stem cells (EpCAM+/CD133+) from malignant ascites in an autologous setting in vitro II.
Figure 5: Catumaxomab eliminates putative cancer stem cells (CD133+/EpCAM+) from malignant ascites in vivo.

The present invention is mainly described herein with respect to the treatment of patients suffering from malignant ascites and the destruction of tumor cells contained in the peritoneal fluid of these patients wherein the tumor cells are associated with EpCAM and CD133. Due to the efficacy shown in the treatment of these patients, it is to be expected that all other kinds of tumors carrying the tumor-associated antigen EpCAM as well as the putative cancer stem cell associated glycoprotein CD133 can be treated in the same effective manner. As EpCAM is a marker for solid tumors, both benign and malign tumors can be efficiently treated. Preferably, these tumors are sarcomas, carcinomas and melanomas. However, any other kind of solid tumors bearing cancer stem cells specified by EpCAM+/CD133+ can be efficiently treated.

Trifunctional bispecific antibodies and different fields of use of these antibodies are known from the prior art. Reference is made in this respect to e.g. DE 198 44 157.6, DE 199 25 586 and EP 1 315 520. Although these state of the art documents describe the use of trifunctional bispecific antibodies, having the three features a, b, and c for anti-tumor immunization, a treatment of tumors by destruction of the tumor cells being associated with both EpCAM antigen and CD133 glycoprotein is not described in these documents. These documents in particular do not disclose that a very specific combination of antigens characterizing the tumor cells as cancer stem cells may be treated by trifunctional bispecific antibodies having the features listed above in a highly efficient and unexpected manner. Furthermore, successful treatment has already been verified in clinical study and by in vitro data.

The pharmaceutical preparation has been designed to achieve at least a destruction of the tumor cells. For this purpose, the pharmaceutical preparation containing the trifunctional antibodies described in more detail below is preferably administered in several doses. The first dose is selected to test a reaction to foreign proteins of the patient in response to the antibodies administered. The second dose is preferably selected to achieve activation and proliferation of the patient's immune cells. A third dose and further doses are selected to particularly achieve destruction of the tumor cells.

Administration of the first dose is carried out in an amount of 1 to 20 µg, preferably 5 to 10 µg of antibody to test for allergic reactions. Administration of the second dose is performed in an amount of 5 to 50 µg, preferably 10-30 µg of antibody. The administration of any further dose is performed in an amount of more than 10 to 500 µg, further preferred 50 to 200 µg of antibody. It should be understood by those skilled in the art that these amounts are only guide numbers which may vary between patients and antibodies. It is in the skills of those working in the medical field, however, to appropriately select the amount of antibody to be employed in individual cases.

The number of applications preferably is an application of one dose 3 to 8 times, preferably 3 to 6 times. The administration is carried out in certain intervals, and preferably intervals of 48 to 72 hours are selected. Preferably, between individual applications an interval of 2-3 days plus/minus several hours is selected. The applications are preferably continued over a period of 10-14 days representing one treatment cycle.

Administration of the primary dose generally is performed in a way that an intraperitoneal or intravenous application form is chosen wherein the antibodies are infused or injected into the patient to achieve a primary immunization, i.e. destruction of the tumor cells. Administration of the dose for secondary and any further immunization generally is carried out by local application, for example intradermally, subcutaneously or intramuscularly. It may also be performed intraperitoneally or intravenously. If a single dose is applied, preferably an intravenous or intraperitoneal route is selected.

As already detailed above, those skilled in the art will choose the respective amount and the site of application to ensure an optimal therapeutic success.

The pharmaceutical preparation provided according to the invention is for example present in an isotonic saline. Other components may be for example stabilizing agents such as Tween^{®} 80 or buffer solutions.

For treatment, intact trifunctional bispecific antibodies are used. The treatment not only achieves the surprising direct destruction of the tumor cells described above but an immunity can also be induced which is directed against the tumor.

Intact antibodies comprise those antibodies having a functional Fc portion. Preferably those are heterologous antibodies, i.e. they are combined of heavy immune globulin chains of different subclasses (combinations, also fragments) and/or different origin (species).

Besides the features a, b, and c described above, in preferred embodiments of the invention the antibodies employed also show the additional features d and e:
d) activates the Fc receptor-positive cell by its binding to the Fc receptor-positive cell whereby the expression of cytokines and/or of co-stimulatory antigens is initiated or increased;
e) the co-stimulatory antigens and/or cytokines transfer to the T cell at least one 2^{nd} activation signal which is required for physiological activation of the T cell, this activation being indicated by an up-regulation of activation markers, a destruction of the tumor cell and/or a proliferation of the T cell.

The antibodies useful according to the present invention are capable of activating the Fc receptor-positive cell whereby the expression of cytokines and/or co-stimulatory antigens is initiated or increased. This avoids any further addition of cytokines and/or co-stimulatory antigens which is an eminent advantage of the invention. Hence, no cytokines and co-stimulatory antigens are added in the present therapeutic method.

By means of the combination and the way of application of the trifunctional antibodies of the present invention, besides a treatment of e.g. the ascites and the pleural effusion or any other solid tumor by direct destruction of the tumor cells also an anti-tumor immunity, preferably a long-lasting anti-tumor immunity can be developed in the patient. The administration is preferably performed in a patient after treatment of the primary tumor, preferably in patients in a minimal residual disease (MRD) situation. In patients with a low amount of residual tumor cells in whom, however, the risk of a recurrency may be high, the application of the pharmaceutical preparation described according to the present invention is especially successful.

The trifunctional bispecific antibodies (trAb) which may be used according to the present invention are known per se. For example they have been described in Lindhofer et al., Blood, 88:4651, 1996; or Lindhofer et al., J. Immunology, 155:219, 1995 or EP 1 315 520.

On the tumor cell, an up-regulation of MHC I as well as an activation of the intracellular processing machinery (proteasome complex) occurs due to the release of cytokines (such as INF-γ and TNF-α) in the direct vicinity of the tumor cell. The cytokines are released due to trifunctional antibody-mediated activation of T cells and accessory cells. This means that due to the intact trAb not only tumor cells are destroyed or phagocytosed but indirectly also the anti-tumor immunogenicity is increased.

Activation of the Fc receptor-positive cell by the trAb is dependent on the subclass or the subclass combination, respectively, of the trAb. As demonstrated in *in vitro* experiments, for example, trAbs of the subclass combination mouse-IgG2a/rat-IgG2b are capable of binding to and simultaneously activating Fc receptor-positive cells resulting in an up-regulation or new formation (expression), respectively, of co-stimulatory antigens such as CD40, CD80, or CD86 on the cell surface of these cells. (Zeidler et al., J. Immunol., 163:1246, 1999). In contrast, bispecific antibodies of the subclass combination mouse-IgG1/rat-IgG2b are able to bind to Fc receptor-positive cells ( Haagen et al., J. Immunology, 1995, 154: 1852-1860) but obviously are unable to activate these cells to a comparable extent ( Gast et al., Cancer Immunol. Immunother., 1995, 40: 390).

While the intact trAb simultaneously binds to and activates the T cell with one binding arm (e.g. to CD3 or CD2), co-stimulatory signals from the Fc receptor-positive cell bound to the Fc portion of the trAb may be transferred to the T cell. This means that only the combination of T cell activation via one binding arm of the trAb and simultaneous transfer of co-stimulatory signals from the Fc receptor-positive cell to the T cell results in an efficient T cell activation. Also, tumor-specific T cells which have been insufficiently activated at the tumor cell and therefore are anergic may be reactivated by the treatment with intact trifunctional antibodies or trispecific antibodies according to the present invention.

Another important aspect in the induction of an anti-tumor immunity is possible phagocytosis, processing and presentation of tumor components by the accessory cells (monocytes/macrophages, or dendritic cells) which have been recruited and activated by the bsab. By this classical mechanism of presentation of antigens both tumor-specific CD4- as well as CD8-positive cells may be generated. Moreover, tumor-specific CD4 cells play an important role in the induction of a humoral immune response in the context of T/B cell cooperation.

Trifunctional bispecific antibodies are able to bind to the T cell receptor complex of the T cell with one binding arm and to tumor-associated antigens on the tumor cell with the second binding arm. Thereby, they activate T cells which destroy the tumor cells by releasing cytokines or by apoptosis-mediating mechanisms. Moreover, there seems to be the possibility that in the frame of activation by trifunctional bispecific antibodies T cells recognize tumor-specific antigens via their receptor and thereby a long-lasting immunization is initiated. Of particular importance in this respect is the intact Fc portion of the trifunctional or trispecific antibody mediating the binding to accessory cells such as monocytes/macrophages and dendritic cells and causing them to become cytotoxic themselves and/or to concomitantly transfer important co-stimulatory signals to the T cell. Obviously, in this manner under certain circumstances a T cell response may be induced against so far unknown tumor-specific peptides.

By redirection of possibly anergized tumor-specific T cells to tumor cells by means of trifunctional bispecific antibodies and simultaneous co-stimulation of such T cells by accessory cells binding to the Fc portion of the antibody the anergy of cytotoxic T cells (CTLs) could be abolished. This means that a T cell tolerance against the tumor existing in the patient may be abolished by means of trifunctional bispecific antibodies and, thus, a long-term anti-tumor immunity may be induced.

Preferably, the antibodies employed according to the present invention are capable of reactivating tumor-specific antigens being in the anergic state. Furthermore, they are capable of inducing tumor-reactive complement-binding antibodies and therefore inducing a humoral immune response.

Binding to the T cell preferably takes place via CD3, CD2, CD4, CD5, CD6, CD8, CD28, CD40L and/or CD44. The Fc receptor-positive cells at least have one Fcγ receptor type I or III.

Antibodies which may be employed according to the present invention are able to bind to monocytes, macrophages, dendritic cells, "natural killer" cells (NK cells) and/or activated neutrophils being Fcγ receptor I and/or III-positive cells (Zeidler, 1999, Zeidler 2000 loc. cit.)

The antibodies which may be employed according to the invention have the effect that the expression of CD40, CD80, CD86, ICAM-1, and/or LFA-3 as co-stimulatory antigens, or/and the secretion of cytokines by the Fc receptor-positive cell is initiated or increased. Preferably, the cytokines are IL-1, IL-2, IL-4, IL-6, IL-8, IL-12, INF-γ and/or TNF-α.

The trifunctional bispecific antibodies which may be employed according to the present invention are for example:
- an anti-CD3 X anti-tumor-associated antigen antibody and/or anti-CD4 X anti-tumor-associated antigen antibody and/or anti-CD5 X anti-tumor-associated antigen antibody and/or anti-CD6 X anti-tumor-associated antigen antibody and/or anti-CD8 X anti-tumor-associated antigen antibody and/or anti-CD2 X anti-tumor-associated antigen antibody and/or anti-CD28 X anti-tumor-associated antigen antibody and/or anti-CD40L X anti-tumor-associated antigen antibody and/or anti-CD44 X anti-tumor-associated antigen antibody;
   most preferably an anti-EpCAMX anti-CD3 antibody is used.

Preferably, the trifunctional bispecific antibody is a heterologous intact rat/mouse bispecific antibody.

By means of the trifunctional bispecific antibodies which may be used according to the present invention, T cells are activated and redirected against the tumor cells. Preferably used trifunctional bispecific antibodies are selected from one or more of the following isotype combinations:
rat-IgG2b/mouse-IgG2a,
rat-IgG2b/mouse-IgG2b,
rat-IgG2b/mouse-IgG3;
rat-IgG2b/human-IgG1,
rat-IgG2b/human-IgG2,
rat-IgG2b/human-IgG3 [oriental allotype G3m(st) = binding to protein A], rat-IgG2b/human-IgG4;
rat-IgG2b/rat-IgG2c;
mouse-IgG2a/human-IgG3 [caucasian allotypes G3m(b+g) = no binding to protein A, in the following indicated as *]
mouse-IgG2a/mouse-[VH-CH1,VL-CL]-human-IgG1-[hinge]-human-IgG3*-[CH2-CH3]
mouse-IgG2a/rat-[VH-CH1,VL-CL]-human-IgG1-[hinge]-human-IgG3*-[CH2-CH3]
mouse-IgG2a/human-[VH-CH1,VL-CL]-human-IgG1-[hinge]-human-IgG3*-[CH2-CH3]
mouse-[VH-CH1,VL-CL]-human-IgG1/rat-[VH-CH1,VL-CL]-human-IgG1-[hinge]-human-IgG3*-[CH2-CH3]
mouse-[VH-CH1,VL-CL]-human-IgG4/rat-[VH-CH1,VL-CL]-human-IgG4-[hinge]-human-IgG4[N-terminal region of CH2]-human-IgG3*[C-terminal region of CH2: > aa position 251]-human-IgG3*[CH3]
rat-IgG2b/mouse-[VH-CH1,VL-CL]-human-IgG1-[hinge-CH2-CH3]
rat-IgG2b/mouse-[VH-CH1,VL-CL]-human-IgG2-[hinge-CH2-CH3]
rat-IgG2b/mouse-[VH-CH1,VL-CL]-human-IgG3-[hinge-CH2-CH3, oriental allotype]
rat-IgG2b/mouse-[VH-CH1,VL-CL]-human-IgG4-[hinge-CH2-CH3]
human-IgG1/human-[VH-CH1,VL-CL]-human-IgG1-[hinge]-human-IgG3*-[CH2-CH3]
human-IgG 1/rat-[VH-CH1,VL-CL]-human-IgG1-[hinge]-human-IgG4[N-terminal region of CH2]-human-IgG3 *[C-terminal region of CH2 : > aa position 251]-human-IgG3*[CH3]
human-IgG 1/mouse-[VH-CH1,VL-CL]-human-IgG1-[hinge]-human-IgG4[N-terminal region of CH2]-human-IgG3 *[C-terminal region of CH2 : > aa position 251]-human-IgG3*[CH3]
human-IgG1/rat-[VH-CH1,VL-CL]-human-IgG1-[hinge]-human-IgG2[N-terminal region of CH2]-human-IgG3*[C-terminal region of CH2 : > aa position 251]-human-IgG3*[CH3]
human-IgG1/mouse-[VH-CH1,VL-CL]-human-IgG1-[hinge]-human-IgG2[N-terminal region of CH2]-human-IgG3*[C-terminal region of CH2 : > aa position 251]-human-IgG3*[CH3]
human-IgG1rat-[VH-CH1VL-CL]-human-IgG1[hinge]-human-IgG3*-[CH2-CH3]
human-IgG1mouse-[VH-CH1VL-CL]-human-IgG1[hinge]-human-IgG3*-[CH2-CH3]
human-IgG2/human-[VH-CH1,VL-CL]-human-IgG2-[hinge]-human-IgG3*-[CH2-CH3]
human-IgG4/human-[VH-CH1,VL-CL]-human-IgG4-[hinge]-human-IgG3*-[CH2-CH3]
human-IgG4/human-[VH-CH1,VL-CL]-human-IgG4-[hinge]-human-IgG4[N-terminal region of CH2]-human-IgG3*[C-terminal region of CH2 : > aa position 251]-human-IgG3*[CH3]
mouse-IgG2b/rat-[VH-CH1VL-CL]-human-IgG1[hinge]-human-IgG3*-[CH2-CH3]
mouse-IgG2b/human-[VH-CH1,VL-CL]-human-IgG1-[hinge]-human-IgG3*-[CH2-CH3]
mouse-IgG2b/mouse-[VH-CH1,VL-CL]-human-IgG1-[hinge]-human-IgG3*-[CH2-CH3]
mouse-[VH-CH1,VL-CL]-human-IgG4/rat-[VH-CH1,VL-CL]-human-IgG4-[hinge]-human-IgG4-[CH2]-human-IgG3*-[CH3]
human-IgG1/rat-[VH-CH1,VL-CL]-human-IgG1-[hinge]-human-IgG4-[CH2]-human-IgG3*-[CH3]
human-IgG1/mouse-[VH-CH1,VL-CL]-human-IgG1-[hinge]-human-IgG4-[CH2]-human-IgG3*-[CH3]
human-IgG4/human-[VH-CH1,VL-CL]-human-IgG4-[hinge]-human-IgG4-[CH2]-human-IgG3*-[CH3]

Most preferably, the isotype combination is mouse IgG2a X rat IgG2b.

The antibodies which may be used according to the present invention preferably are monoclonal, chimeric, recombinant, synthetic, semi-synthetic, or chemically modified intact antibodies having for example Fv, Fab, scFv, or F(ab)₂ fragments.

Preferably, antibodies or derivatives or fragments of humans are used, or those which have been modified to be suitable for the use in humans (so-called "humanized antibodies") (see for example Shalaby et al., J. Exp. Med. 175 (1992), 217; Mocikat et al., Transplantation 57 (1994),405).

The preparation of the different types of antibodies and antibody fragments mentioned above is well-known to the skilled artisan. The preparation of monoclonal antibodies, preferably of those originating in mammals, e.g. human, rat, mouse, rabbit, or goat, can be performed using conventional methods as those described for example in Köhler und Milstein (Nature 256 (1975), 495), in Harlow and Lane (Antibodies, A Laboratory Manual (1988), Cold Spring Harbor) or in Galfré (Meth. Enzymol. 73 (1981), 3) or in DE 195 31 346.

It is further possible to prepare the antibodies described by means of recombinant DNA technology according to techniques known to those skilled in the art (see Kurucz et al., J. Immunol. 154 (1995), 4576; Hollinger et al., Proc. Natl. Acad. Sci. USA 90 (1993), 6444).

The preparation of antibodies having two different specificities, the so-called trifunctional bispecific antibodies, can be performed on the one hand using recombinant DNA technology but on the other hand also by the so-called hybrid hybridoma fusion technique (see for example Milstein et al., Nature 305 (1983), 537). For this purpose, hybridoma cell lines each producing antibodies having one of the desired specificities are fused and cellular clones (quadromas) producing antibodies having both specificities are identified and isolated.

The problem underlying the present invention can be solved using trifunctional bispecific antibodies which exhibit the properties and effects characterized. In the following, the preparation of antibodies showing two and three specificities is described in more detail. To provide such bispecific and trispecific antibodies is known from the prior art, and references describing such techniques of preparation are incorporated herein by reference in their entirety.

According to the present invention there are for example used trifunctional bispecific antibodies. Trifunctional bispecific antibodies are composed of two antibody semi-molecules (each having a H and a L immunoglobulin chain) each of which represents one specificity which additionally like normal antibodies have an Fc portion performing the well-known effector functions. They are preferably prepared using the quadroma technology. This method of preparation is described exemplarily in DE-A-44 19 399. For complete disclosure this document is incorporated by reference in its entirety also with respect to a definition of bispecific antibodies. It should be understood, however, that also other methods of preparation may be employed if they lead to the trifunctional bispecific antibodies according to the above definition which are required by the present invention.

For example, trifunctional bispecific antibodies may be produced in a sufficient amount using a newly developed method of preparation (Lindhofer et al., J. Immunology 1995, 155: 219). The combination of 2 trifunctional bispecific antibodies directed against 2 different tumor-associated antigens (e.g. c-erb-B2, and Ep-CAM) on the mammary carcinoma cells minimizes the risk that tumor cells expressing only one antigen would remain unrecognized.

Further advantages of trifunctional bispecific antibodies having the ability of redirecting T cells over the above-mentioned bsF(ab')2 fragments may be detailed as follows:
1. It is possible for Fc receptor-positive cells to bind to intact trAbs and to contribute on the one hand directly to tumor destruction via ADCC (antibody-dependent cell-mediated cytotoxicity) and on the other hand to T cell activation as explained in more detail above.
2. By intact T cell-redirecting trAbs also anergized tumor-specific T cells are recruited to the tumor cell which according to the invention may be reactivated directly at the tumor site. This may not be achieved by an bsF(ab')2 fragment having the specificities anti-CD64 X anti-tumor-associated antigen.

Binding of the trAb to Fcγ-RI has two essential advantages with regard to an optimal anti-tumor effectiveness:
(1) Fcγ-RI-positive cells have the ability to eliminate tumor cells by ADCC and, thus, are able to contribute synergistically to the anti-tumor effect of the cytotoxic T cells recruited to the tumor cell by the trAb.
(2) FcγRI-positive cells (such as monocytes/macrophages/ dendritic cells) are able to provide important co-stimulatory signals similar to those involved in antigen presentation to the T cell and, thereby, to prevent anergizing of the T cell. Furthermore, because of the intact trAb-mediated interaction of T cell with accessory cell and tumor cell there can be stimulated as a desired by-product even T cells the T cell receptor of which recognizes tumor-specific peptides (presented via MHC antigens on the tumor cell). In this constellation, the co-stimuli necessary for correct activation of the T cell would be provided by the accessory cell (such as the monocyte). Thus, besides the direct, T cell receptor-independent trAb-mediated tumor destruction the antibody of the present invention should also be able to activate and generate tumor-specific T cells which after degradation of the trAb continue to patrol in the patient. This means that similar to gene-therapeutical approaches (e.g. by incorporation of co-stimulatory antigens such as B-7 into the tumor cell) the tumor tolerance in the patient may be abolished by means of intact trAbs.

In the following examples, which refer to the enclosed figures, the invention is described in more detail. However, the example is only illustrative for the present invention, and the invention is not limited to the specific example but can be varied within the scope of the claims.

### Example 1:

The pancreatic tumor cell line CAPAN has a cancer stem cell like expression profile expressing CD133 and EpCAM on the surface. Experiments showed a complete elimination of CD133+/EpCAM+ CAPAN cells by the trifunctional antibody catumaxomab in concentrations as low as 0,5 ng/ml, but revealed only minimal (max 30%) antibody-induced killing of CAPAN cells with an anti-EpCAM monoclonal antibody (figure 2).

### Example 2:

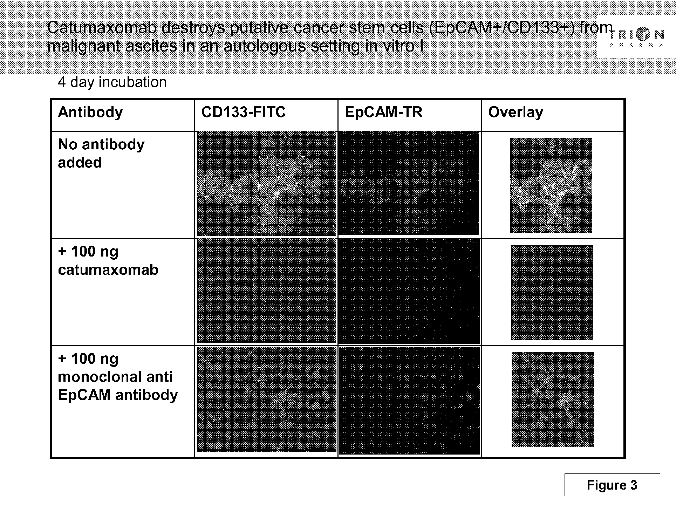

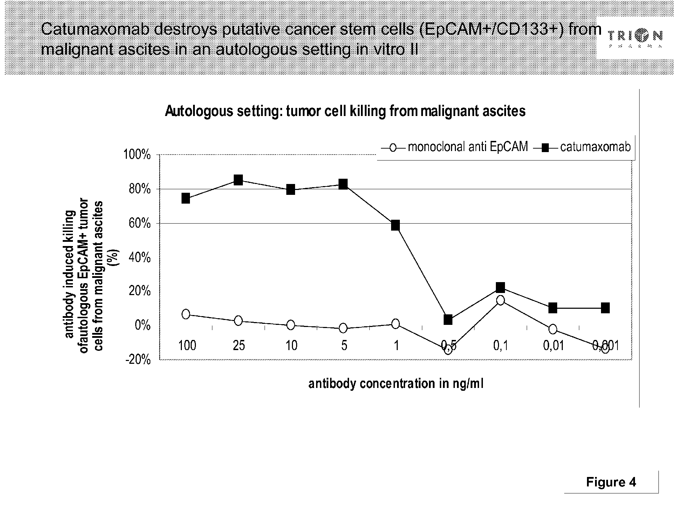

Furthermore, in in vitro experiments with an ascites cell preparation, we also detected a CD133+/EpCAM+ population which was again destroyed completely by catumaxomab but remained unaffected by a monoclonal anti-EpCAM antibody. The clear superiority of the trifunctional antibody was shown with immunofluorescence doublestaining for CD133 and EpCAM and in a XTT potency assay (figure 3 and 4).

### Example 3:

Cytospin preparations from malignant ascites patients participated in a pivotal clinical study (IP-REM-AC-01) with catumaxomab were analysed regarding the presence of CD133+/EpCAM+ putative CSCs in MA and, more importantly, the elimination of this cell population from the peritoneal fluid by means of catumaxomab therapy.

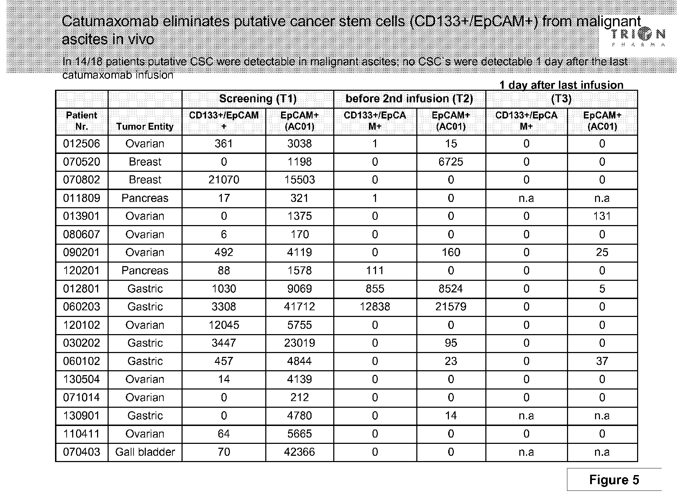

Before therapeutic intervention, CD133+/EpCAM+ cells were detected in the peritoneal fluids of 14 from 18 patients (78%) suffering from MA. After the 1st infusion of catumaxomab (10µg), 9 of these 14 patients (64%) showed complete elimination of the CD133+/EpCAM+ cells. After 4 i.p. catumaxomab infusions (10µg day 0, 20µg day 3, 50µg day 7 and 150µg day 10) the CD133+/EpCAM+ cells were completely eliminated from the peritoneal fluids of all 14 MA patients (100%) (figure 5).

Consequently, catumaxomab-based therapeutic measures may offer an additional treatment opportunity to eliminate CSCs in EpCAM+ malignancies.

### METHODS

### 1.1 Cell lines and antibodies

As target cell line for the XTT potency assay the EpCAM and CD133 positive pancreas carcinoma cell line CAPAN (ATCC HTB-79) was used. CAPAN cells have a cancer stem like character expressing both EpCAM and the postulated cancer stem cell marker CD133. PBMC from healthy donors served as effector cell populations. The trifunctional antibody catumaxomab (anti-EpCAM x anti-CD3; TRION Pharma, Munich) targets with one arm the EpCAM molecule at the surface of tumor cells, recruits with the other arm CD3+ T cells and attracts accessory cells via its potent Fc region. As control antibodies the monoclonal antibody HO-3 (anti-EpCAM, TRION Research, Martinsried).

### 1.2 Isolation of PBMC from peripheral blood

Blood from a healthy donor was purified using density centrifugation through Ficoll Histopaque (PAN Biotech, Aidenbach, Germany) at 897 x g, 15 min, 10 °C, acceleration 2 and brake 0. Mononuclear cells were washed twice with PBS without Mg²⁺ or Ca²⁺ (PAN Biotech, Aidenbach, Germany) and centrifuged at 458 x g, 10 min, 20 °C. The supernatant was removed and the pellet was resuspended in 20 ml RPMI 1640, supplemented with 2 mM glutamine, 1 mM sodium pyruvate and 1 mM non-essential amino acids and 10% FCS (PAN Biotech, Aidenbach, Germany). Cell number and viability were determined by trypan blue staining (Sigma, Deisenhofen, Germany).

### 1.3 XTT Potency assay design

The XTT potency assay was performed according to the manufacturers' manual (Roche Diagnostics, Mannheim). In brief, PBMCs were used in a concentration of 1x10e5 cells/well and CAPAN cells were used in a concentration of 1x10e4 cells/well which represents an E:T ratio of 10:1. Catumaxomab and HO-3 were used at concentrations ranging from 100 to 0,001 ng/ml.

The autologous XTT potency assay was performed with an ascites cell preparation freshly harvested from a patient's malignant ascites. 2x10e5 ascites cells were seeded in 96 well plates and catumaxomab or H0-3 were added in concentrations ranging from 100 to 0,001 ng/ml.

After 4 days incubation, PBMC were discarded and the proliferation of tumor cells was analysed using the XTT Cell Proliferation Kit II (Roche Diagnostics, Mannheim). Extinction was measured in a Versamax microplate reader (Molecular Devices) and raw data were analysed using Excel XP (Microsoft, USA).

### 1.4 Immunofluorescence

CAPAN cells or ascites cells were harvested by centrifugation or ficoll density centrifugation if contaminated by erythrocytes and spinned on cytospin slides with a concentration of 2,5 x 10e5 cells per slide for tumor cell counts. Slides were dryed and stained for tumor cells. Double-Staining of CAPAN cells and ascites cell preparation was done with an anti CD133 Ab and its corresponding secondary antibody anti mouse IgG1 Alexa Fluor 488 (FITC, Invitrogen) and a directly Texas Red (Alexa Fluor 594, Invitrogen) labelled anti- EpCAM antibody HO-3 (TRION Research).

### 1.5 Patient population

18 CTX-refractory patients with MA caused by a variety of primary carcinoma diseases (i.e. ovarian, pancreas and gastric cancer) were analyzed for the presence CD133+/EpCAM+ cells in peritoneal fluids by means of CD133+/EpCAM+ double staining on cytospin preparations. Analyses were performed before, 2 days after the first and 1 day after the last catumaxomab infusion, respectively. Double stained cytospin preparations were stained and evaluated with a computerized image analysis system according to 1.4.

## Claims

1. Use of a trifunctional bispecific antibody having the following properties of:
a) binding to a T cell;
b) binding to EpCAM as tumor-associated antigen on a tumor cell wherein the tumor cell additionally carries the membrane bound glycoprotein CD133;
c) binding by its Fc portion to Fc receptor-positive cells
for the preparation of a pharmaceutical composition for the destruction of cancer stem cells carrying the tumor-associated antigen EpCAM and the membrane bound glycoprotein CD133 in a population of patients suffering from a tumor.

2. The use according to claim 1
**characterized in that**
said tumor is a solid tumor, either benign or malign, and optionally selected from the group consisting of sarcomas, carcinomas and melanomas.

3. The use according to claim 1 or 2,
**characterized in that**
said pharmaceutical preparation is adapted for the administration in the form of a first dose and of at least one further dose, optionally
said pharmaceutical preparation is adapted for the administration in the form of a second dose for the activation and proliferation of the immune cells and of at least a third dose for the destruction of the tumor cells.

4. The use according to one or more of the preceding claims,
**characterized in that**
said pharmaceutical preparation is adapted for the administration in the form of a first dose to test for allergic reactions against the antibody administered.

5. The use according to one or more of the preceding claims,
**characterized in that**
said pharmaceutical preparation is adapted to contain for the administration on the first day a first dose of 1 to 20 µg, preferably 5 to 10 µg of the antibody, for the administration of the second dose an amount of 5 to 50 µg, preferably 10 to 30 µg of the antibody, and for the administration of any further dose an amount of 10 to 500 µg, preferably 50 to 200 µg of the antibody.

6. The use according to one or more of the preceding claims,
**characterized in that**
said pharmaceutical preparation preferably is adapted for an intraveneous or intraperitoneal application for primary immunization and is adapted for an intraveneous, intraperitoneal, intradermal, subcutaneous, or intramuscular application for secondary immunization.

7. The use according to one or more of the preceding claims,
**characterized in that**
said antibody is capable of binding to Fc receptor-positive cells having an Fcγ receptor I or III.

8. The use according to one or more of the preceding claims,
**characterized in that**
said antibodies are capable of binding to monocytes, macrophages, dendritic cells, natural killer cells (NK cells) and/or activated neutrophil cells as the Fcγ receptor I- and/or III-positive cells, and furthermore optionally are selected to increase the secretion of the cytokines IL-1, IL-2, IL-4, IL-6, IL-8, IL-12, INF-γ and/or TNF-α.

9. The use according to one or more of the preceding claims,
**characterized in that**
said bispecific antibody is selected to be an anti-CD3 X anti-tumor-associated antigen antibody and/or anti-CD4 X anti-tumor-associated antigen antibody and/or anti-CD5 X anti-tumor-associated antigen antibody and/or anti-CD6 X anti-tumor-associated antigen antibody and/or anti-CD8 X anti-tumor-associated antigen antibody and/or anti-CD2 X anti-tumor-associated antigen antibody and/or anti-CD28 X anti-tumor-associated antigen antibody and/or anti-CD40L X anti-tumor-associated antigen antibody and/or anti-CD44 X anti-tumor-associated antigen antibody, preferably an anti EpCAM X anti-CD3 antibody.

10. The use according to one or more of the preceding claims,
**characterized in that**
said trifunctional antibody is selected from a heterologous rat/mouse bispecific antibody, wherein optionally said trifunctional antibody is selected from at least one member of the following group of isotype combinations in its Fc-region:
rat-IgG2b/mouse-IgG2a,
rat-IgG2b/mouse-IgG2b,
rat-IgG2b/human-IgG1,
mouse-[VH-CH1,VL-CL]-human-IgG1/rat-[VH-CH1,VL-CL]-human-IgG1-[hinge]-human-IgG3*-[CH2-CH3]
[* = caucasian allotypes G3m(b+g) = no binding to protein A]

11. Use according to one or more of the preceding claims, wherein said trifunctional antibody is administered in an amount of 0.05-15 µg/kg body weight.

12. Use of a trifunctional bispecific antibody according to one or more of the preceding claims, wherein said antibody has the following properties of:
a) binding to a T cell;
b) binding to EpCAM as tumor-associated antigen on a tumor cell associated with malignant ascites and/or pleural effusion wherein the tumor cell additionally carries the membrane bound glycoprotein CD133;
c) binding by its Fc portion to Fc receptor-positive cells
for the preparation of a pharmaceutical composition for the treatment of malignant ascites and/or pleural effusion for the destruction of tumor cells in the ascites fluid and/or in the pleural effusion in a sub-population of patients carrying on said tumor cell EpCAM as tumor-associated antigen and additionally the membrane bound glycoprotein CD133.
